# EUROPEAN PATENT SPECIFICATION

(11) **EP 2 277 994 B1**
(45) Date of publication and mention of the grant of the patent: **21.02.2018**
(21) Application number: 09725611.9
(22) Date of filing: 17.03.2009
(51) Int. Cl.: C12N 5/073, C12N 5/02, C12R 1/91

(54) **METHOD FOR PRODUCING FIBROPLAST CELLS FROM THE NEWBORN'S NAVEL-CORD**
VERFAHREN ZUR HERSTELLUNG VON FIBROBLASTEN AUS DER NABELSCHNUR VON NEUGEBORENEN
PROCÉDÉ DE FABRICATION DE CELLULES DE TYPE FIBROBLASTES À PARTIR DU CORDON OMBILICAL DE NOUVEAU-NÉS

(30) Priority: 27.03.2008 RU 2008111708
(43) Date of publication of application: 26.01.2011
(73) Proprietor: North State Ltd., Tortola (VG)
(72) Inventor: PRIKHOD'KO, Aleksandr Viktorovich, Moskovskaya obl. 141002 (RU); ISAEV, Artur Aleksandrovich, Moscow 119607 (RU); KISELJOV, Sergejj L'vovich, Moscow 123458 (RU); LAGAR'KOVA, Marija Andreevna, Moscow 105318 (RU); KOSHELEVA, Nastas'ja Vladimirovna, Moscow 121099 (RU); SABURINA, Irina Nikolaevna, Moscow 119607 (RU); MELIKHOVA, Varvara Sergeevna, Moscow 125362 (RU)
(74) Representative: Glawe, Delfs, Moll
(86) International application number: PCT/RU2009/000128
(87) International publication number: WO 2009/120111

(56) References cited:
- WO-A1-2006/019357
- WO-A2-02/092794
- WO-A2-2008/019148
- RU-C1- 2 308 957
- NANAEV A K ET AL: "STROMAL DIFFERENTIATION AND ARCHITECTURE OF THE HUMAN UMBILICAL CORD", PLACENTA, LONDON, GB, vol. 18, no. 1, 1 January 1997 (1997-01-01), pages 53-64, XP008029778, DOI: 10.1016/S0143-4004(97)90071-0
- LU LU-LU ET AL: "Isolation and characterization of human umbilical cord mesenchymal stem cells with hematopoiesis-supportive function and other potentials", HAEMATOLOGICA, THE HEMATOLOGY JOURNAL : OFFICIAL ORGAN OF THE EUROPEAN HEMATOLOGY ASSOCIATION, FONDAZIONE FERRATA STORTI, ROME, IT, vol. 91, no. 8, 1 August 2006 (2006-08-01) , pages 1017-1026, XP009110905, ISSN: 0390-6078
- CAN ALP ET AL: "Concise review: human umbilical cord stroma with regard to the source of fetus-derived stem cells", STEM CELLS, vol. 25, no. 11, 27 July 2007 (2007-07-27) , pages 2886-2895, XP002521370, ISSN: 1549-4918, DOI: 10.1634/STEMCELLS.2007-0417 [retrieved on 2007-08-09]
- ROMANOV Y A ET AL: "Searching for alternative sources of postnatal human mesenchymal stem cells: Candidate MSC-like cells from umbilical cord", STEM CELLS, ALPHAMED PRESS, DAYTON, OH, US, vol. 21, no. 1, 1 January 2003 (2003-01-01), pages 105-110, XP002331940, ISSN: 1066-5099, DOI: 10.1634/STEMCELLS.21-1-105
- JAFFE E A ET AL: "CULTURE OF HUMAN ENDOTHELIAL CELLS DERIVED FROM UMBILICAL VEINS IDENTIFICATION BY MORPHOLOGIC AND IMMUNOLOGIC CRITERIA", JOURNAL OF CLINICAL INVESTIGATION, AMERICAN SOCIETY FOR CLINICAL INVESTIGATION, US, vol. 52, no. 11, 1 January 1973 (1973-01-01), pages 2745-2756, XP008029776, ISSN: 0021-9738, DOI: 10.1172/JCI107470
- DERYL L. TROYER ET AL: "Concise Review: Wharton's Jelly-Derived Cells Are a Primitive Stromal Cell Population", STEM CELLS, vol. 26, no. 3, 1 March 2008 (2008-03-01), pages 591-599, XP055059242, ISSN: 1066-5099, DOI: 10.1634/stemcells.2007-0439
- STENN K S ET AL: "DISPASE, A NEUTRAL PROTEASE FROM BACILLUS POLYMYXA, IS A POWERFUL FIBRONECTINASEAND TYPE IV COLLAGENASE", JOURNAL OF INVESTIGATIVE DERMATOLOGY, NATURE PUBLISHING GROUP, GB, vol. 93, no. 2, 1 August 1991 (1991-08-01) , pages 287-290, XP001070905, ISSN: 0022-202X, DOI: 10.1111/1523-1747.EP12277593
- SCHUGAR REBECCA C ET AL: "High harvest yield, high expansion, and phenotype stability of CD146 mesenchymal stromal cells from whole primitive human umbilical cord tissue", JOURNAL OF BIOMEDICINE AND BIOTECHNOLOGY, DAR AL-NASR AL-ILIKTRUNI, CAIRO, EG, vol. 2009, 789526, 11 September 2009 (2009-09-11), pages 1-11, XP009150092, ISSN: 1110-7243 [retrieved on 2009-12-16]
- ROMANOV Y. A. ET AL.: 'Searching for Alternative Sources of Postnatal Human Mesenchymal Stem Cell: Candidate MSC-Like Cells from Umbilical Cord' STEM CELLS vol. 21, no. 1, 2003, pages 105 - 110, XP002331940

## Description

### Field of the Invention

The invention relates to biotechnology namely to a method for producing fibroblast-like cells from the umbilical cord and their use thereof. The fibroblasts produced might be widely used in medical practice.

At present the umbilical cord is considered as a source for producing several types of cells including human umbilical cord vein endothelial cells (HUVEC) and fibroblast cells (FPC). HUVECs are widely used by a lot of research groups worldwide. In pharmacological, pharmacokinetic and other investigations endothelial cells that are nonpassaged are used as a rule.

### Background of the Invention

There is a method for obtaining allogeneic fibroblasts from the newborn umbilical cord after a normal deliver, when a fragment of the umbilical cord is used. The veins of the umbilical cord are cannulated on the both sides and washed out with a Hank's solution at first, then with a 0.1 % type I collagenase solution on the DMEM medium. Then the fragment is incubated at the temperature 37° C for 30 minutes and finally the cord tissues are blended. The separated cells are collected by washing them out with the Hank's solution. The resulting cell suspension is centrifuged at 1000 r/min for 5 minutes to get a cell residue which is later re-suspended within a DMEM growth medium containing 10% of a bovine serum, 100 U/ml penicillin, 100 U/ml streptomycin, 2 mM glutamine, 1 mM sodium pyruvate, 10 ng/ml of the basic fibroblast growth factor bFGF. The cells residue re-suspended within the DMEM growth medium is moved to culture plates and cultured changing the DMEM growth medium twice a week until a monolayer is formed. When the monolayer is formed the cells are re-seeded in the ratio 1:3. The given method is described in RU 2308957 of October 27, 2007 and we accepted it as the closest analogue. The method has disadvantages such as a low yield of fibroblast cells, as well as obtaining only fibroblast cells of the umbilical cord vein that limits their further differentiation capabilities and usage.

### Brief Description of the Figures

**Fig.1** The culture of fibroblast-like cells after the 2^{nd} and the 4^{th} passages.
**Fig. 2** The vimentin expression in the cells after the 2^{nd} and the 4^{th} passages.
**Fig. 3** The primary culture of HUVECs on the 7^{th} culturing day
**Fig. 4** The expression of the type I collagen in cells
**Fig. 5** The expression of the type IV collagen in cells after the 4th passage.

### Detailed Description of the Invention

The invention is set out in the appended claims. The embodiments of the description which do not fall within the scope of said claims are provided for illustrative purposes only and do not form part of the present invention.

We aimed to develop an easy method for producing fibroblast-like cells from the entire tissue of the umbilical cord allowing to isolate both fibroblasts from the vein and mesenchymal, dermal and stromal fibroblast cells.

As a result of our work the method for producing fibroblast-like cells from the newborn umbilical cord is proposed, which comprises the following stages:
1. preparing a fragment of the umbilical cord;
2. homogenizing the prepared fragment;
3. processing the homogenate with a solution containing the enzymes, type I collagenaze and type IV collagenase, in the ratio of 1:1; the proportion of the homogenate to the enzyme solution is 1:5;
4. filtering and centrifuging the filtrate to produce the residue of fibroblast-like cells.

The proposed method for producing fibroblast-like cells allows not only to increase the yield of fibroblasts thereby to provide the effective usage of an expensive biomaterial, the umbilical cord, but also to produce a wide range of embryonic fibroblast-like cells such as mesenchymal, dermal and stromal fibroblasts. A wide range of the cells isolated allows their further differentiation into mesenchymal cells, such as cardiomyocytes, for example. Stromal fibroblasts may further differentiate into cartilage, muscle, bone and other cells such as neuronal or insulin-producing cells under the influence of additional factors. Embryonic dermal fibroblast-like cells may differentiate into keratinocytes and dermal fibroblasts. Thus, the method claimed and the cells produced may be applied to produce the above cells.

A stage of preparing a fragment of the umbilical cord can involve additionally an isolation of endothelial cells from the umbilical cord vein. With that two and more cell types may be produced from the same sample.

### The Way of the Method Implementation

The human umbilical cord is obtained in maternity hospitals after a normal delivery with an informed consent of a voluntary obstetric patient.

A biomaterial is collected in compliance with all the aseptic and antiseptic regulations having made an obligatory blood test for HIV, RW and hepatitis B and C markers and when the test results are negative.

A contamination with any agent is the contraindication for collecting a biomaterial.

All manipulations on the biomaterial are carried out in the laboratory designed to work only with human biomaterial.

The work with human biomaterials has to be done in compliance with the recommendations "Regulations for sterility control of the preserved blood, blood components, preserved bone marrow products, volume expanders and preserving solutions" approved by the Public Health Ministry (Minzdrav) # 4-42-4-85 of 17, September 1985.

All manipulations on biomaterials are made under sterile conditions of a laminar flow cabinet of the class II biosafety.

The preparation of a fragment of the umbilical cord involves the following manipulations.
1. Washing off the blood and mucus clots with Hank's solution.
2. Washing out the umbilical cord vein by a syringe with Hank's solution, an antibiotic and fungizone.
3. Cutting the umbilical cord into pieces of 2 cm long. Every piece is cut longwise and washed out with Hank's solution.

An isolation of fibroblast-like cells from the umbilical cord involves the following manipulations:
1. The prepared fragments of the umbilical cord are homogenized.
2. The resulting mass is put into tubes, a combination of working solutions of collagenase I and collagenase IV (the enzyme ratio is 1:1) is added in the proportion of 1:5.
3. The biomaterial is fermented while mixing up the tube content at intervals. The period of incubating depends on the enzyme concentration in a solution and their activity.
4. Having been fermented the suspension is filtered via a double sieve to clear away remaining non-fermented tissue pieces and a collagen excess.
5. After filtering the resulting suspension is put into centrifuge tubes and centrifuged to obtain the fibroblast residue.

It is reasonable to isolate endothelial cells while preparing the umbilical cord fragment. To do this, additional manipulations can be carried out after washing the umbilical cord off.
1. One end of the cord is fixed, the vein is filled with a ferment solution (a dispase solution - 2.5 mg/ml). The second end is fixed as well to control the vein air-tightness.
2. Then the sample is incubated in CO₂ for 15 minutes.
3. To facilitate the enzyme access to every spot of the vein wall the cord is massaged.
4. The vein content is washed out twice with a sterile Hank's solution and then with a growth medium.
5. The produced suspension of the cells is pipetted and then centrifuged to obtain the endothelial cells residue.

The feasibility of the method can be confirmed by the following examples.

### Example 1. Isolation of Fibroblast-Like Cells.

Under sterile conditions of the laminar flow cabinet the delivered umbilical cord (a fragment of 7-10 cm long) is moved from a container into a Petri plate (made in Russia) filled with Hank's solution (manufactured by *PanEco* or similar) adding an antibiotic and fungizone. Blood and mucus clots are washed off the biomaterial with Hank's solution (manufactured by *PanEco* or similar) changing the solution three times. Having detected the location of the umbilical cord vein, its ends are widen with a sterile forceps and a 2 ml syringe tip filled with Hank's solution is inserted into the vein. The vein is washed out twice with the sterile Hank's solution (manufactured by *PanEco* or similar) containing an antibiotic and fungizone. The umbilical cord is cut into pieces of 2 cm long. Every piece is cut longwise, washed out with Hank's solution and homogenized. The produced mass is put into two 15 ml centrifuge tubes (or into one 50 ml tube), adding a mixture of 0.075% working solutions of collagenase I and collagenase IV (1:1) in the proportion of 1:5. Working solutions are prepared by diluting enzymes in a liquid serum- and glutamine-free DMEM growth medium (manufactured by *PanEco* or similar).

The biomaterial is fermented for 8-10 hours at 37° C mixing up the tube content at intervals. The efficiency of enzyme processing is evaluated by changing the degree of a suspension viscosity. Incubating must be discontinued after the viscosity has significantly increased. The moment of sharp increase of the viscosity is detected visually.

Having been fermented the suspension is filtered through a double sieve to clear away the remaining non-fermented tissue parts and a collagen excess. The tube and the sieve are washed out with Hank's solution (manufactured by *PanEco* or similar). The suspension produced after filtering is put into other 15 ml centrifuge tubes (or a 50ml centrifuge tube) and is centrifuged for 15 minutes at 1000 r/min, g=90. As a result the residue of fibroblast-like cells is obtained.

### Example 2. Sequential Isolation of Endothelial and Fibroblast-Like Cells.

The manipulation succession is carried out as described in Example 1. Additionally, having washed out the umbilical cord vein, its one end is fixed with corn tongs. Holding the sample carefully the vein is filled up with an enzyme solution (a dispase solution - 2.5 mg/ml). The solution is prepared by diluting an enzyme with a serum- and glutamine-free growth medium. The other end of the vein is also fixed to control the vein air-tightness. The sample is placed into a Petri plate (made in Russia) and incubated in CO₂ for 15 minutes. Every 5 minutes the cord is massaged to facilitate an enzyme access to every spot of the vein wall. In 15 minutes the sample is moved to the laminar, the corn tongs removed, the vein content washed out twice with the sterile Hank's solution (manufactured by *PanEco* or similar), and then with a growth medium. The cell suspension produced is pipetted and centrifuged for 5 minutes at 1000 r/min. The residue of endothelial cells is obtained.

Having isolated the endothelial cells, the fragment of the umbilical cord is placed into another Petri plate with Hank's solution (manufactured by *PanEco* or similar), an antibiotic and fungizone. Then the fragment is washed out in Hank's solution, changing the solution twice. The umbilical cord fragment is cut into pieces of 2 cm long. The fibroblast cell isolation is carried out as described in Example 1.

It must be noted that the duration of enzyme processing depends on the enzyme concentration in a solution, the activity of enzyme products of different manufacturers and a number of other factors. That the manipulation on a sample is discontinued in the time of visually detectable sharp increase in a suspension viscosity is remarkable.

### Example 3. Culturing Fibroblasts and Endothelial Cells.

The cell residues obtained after centrifuging are re-suspended in a growth medium, the number of the cells isolated is counted in the Goryaev camera. The suspensions produced are placed into Petri plates (*Greiner* or *Coming*).

To culture fibroblast-like cells isolated from the umbilical cord any appropriate growth medium may be used, for example, a medium of the following composition: a liquid DMEM/F12 medium in the proportion 1:1 *(PanEco* or similar), 10% of a Fetal Clone 1 serum (*Hyclone* or similar), 1X ITS (*Hyclone* or similar), 1 ng/ml of bFGF (*Chemicon* or similar), 5 U/ml heparin (*PanEco* or similar), 50 mg/ml gentamicin (*PanEco* or similar). All the components are calculated at 11 of a DMEM/F12 medium.

To culture endothelial cells isolated from the umbilical cord any appropriate growth medium may be used, for example, a medium of the following composition: a 199 medium with Erl salts (*PanEco* or similar), 20% of a Fetal Clone 1 serum (*Hyclone* or similar), 1% of an antibiotic (*PanEco* or similar), 2.5 ng/ml of an epidermal growth factor (*Chemicon* or similar), a HEPES solution (*PanEco* or similar), a 7% solution of NaHCO₃ (*PanEco* or similar).

Endothelial cells are seeded at the density of 5x10³ cells/ cm², fibroblast-like cells - at the density of 10⁴ cells/ cm².

The cells are cultured under standard conditions: at 37° C and 5% CO₂, changing the medium every 2-3 days. The cultures are passaged when they achieve 70-80% confluency. Before passaging the medium is poured out, the plate is washed out with a Versen solution (*PanEco* or similar) three times. The plate content is immersed in a 0.25% trypsin solution for 4-7 minutes. Having stirred well the cell suspension is dispensed in other Petri plates (*Greiner* or *Coming)* at the density of 8,000-15,000 cells/cm². The first passaging is carried out in 12-14 days after seeding. Later, the amount of fibroblast-like cells doubles every 48-72 hours. Correspondingly, starting in the second passage 2 - 3 days elapse between passages.

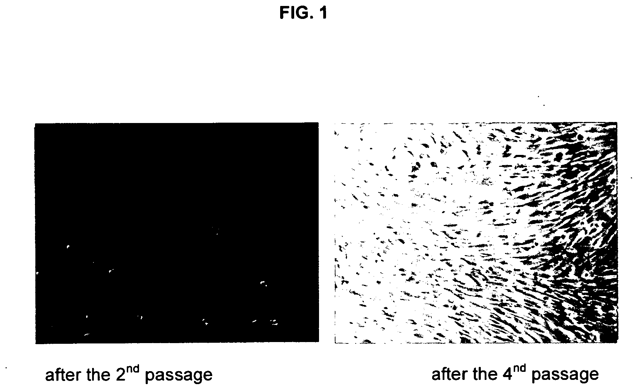

In a culture fibroblast-like cells grow only when attached to a culture dish surface (Petri plates or culture vials). During all the culturing time they maintain a diploid karyotype and a high proliferative activity for 4-6 passages (Fig.1).

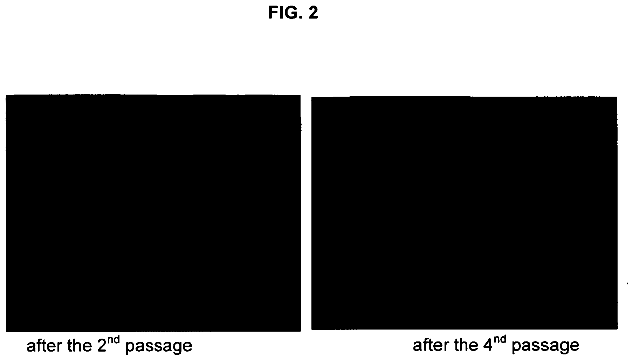

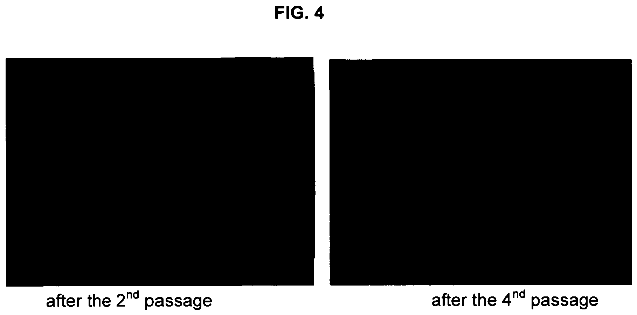

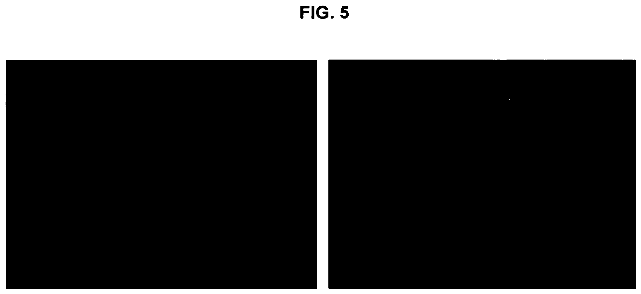

Cultured fibroblast-like cells, isolated from the human umbilical cord express vimentin, collagen of I and IV types (Fig.2, 4, 5), alpha-actin and others (chondroitin sulfate, elastin, fibronectin, keratin 18, keratin 19, heparin sulfate), but they do not express CD45, CD11b, CD14 and have a low expression of histo-compatibility antigens.

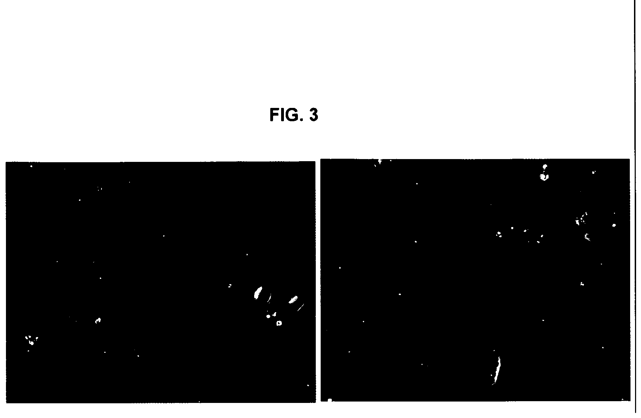

As a rule, non-passaged endothelial cells of the umbilical cord vein are used. The endothelial cells grow in a culture only when they are attached to a culture dish surface (Petri plates or culture vials) and have a phenotype typical for endotheliocytes (Fig.3).

### Example 4. Cryoconservation of Fibroblasts.

After the culture of fibroblast-like cells has achieved the fourth passage the cells are cryoconserved in cryovials in the amount of 10 mln.

To eliminate the risk of possible bacterial and viral contaminations before cryoconserving a part of the cells undergoes a thorough bacterial and viral control (product contamination control), the rest of the cells are cryoconserved.

The cells undergo testing for:
- bacterial contamination,
- fungal contamination,
- presence of hepatitis B virus DNA,
- presence of hepatitis C virus RNA,
- presence of HIV-1 proviral DNA,
- presence of HIV-2 proviral DNA,
- presence of HSV-1 DNA,
- presence of HSV-2 DNA,
- presence of cytomegalovirus DNA,
- presence of toxoplasma DNA,
- presence of mycoplasma DNA.

For cryoconservation a conditioned medium is selected from Petri plates with a confluent monolayer culture. The cell culture is washed out with a Versen solution three times and trypsinized at 37° C and 5% CO₂ for 10 minutes. The resulting suspension is homogenized, the cells are counted in the Goryaev camera. The homogenate is centrifuged for 10 minutes at 1000 r/min. The supernatant is removed, the cells re-suspended in a cryoconservation medium (a FetalClone 1 human umbilical cord blood serum and 7% dimethyl sulfoxide) in the concentration of 10 mln cells per 1 ml cryoconservation medium. The cell suspension is put into 2 ml cryovials with a 5 ml pipette. The cryovials are labeled according to the standard pattern. The cells undergo controlled-rate freezing in a special freezer up to - 80° C and then placed into Dewar vessels with liquid nitrogen in a cryostorage facility. Up to 200 mln fibroblast-like cells can be isolated from a 7-10 cm umbilical cord at the fourth passage. These 200 mln fibroblast cells can be obtained and cryoconserved in approximately 25-30 days after receiving the biomaterial in a laboratory.

## Claims

1. A method for producing fibroblast-like cells from newborn's umbilical cord, wherein said method comprises the following steps:
a) preparing a fragment of the human umbilical cord,
b) homogenizing the prepared fragment,
c) fermentation of the homogenate with a working solution containing type I collagenase and type IV collagenase in a ratio of the enzymes of 1:1 at a proportion of the homogenate to the enzyme working solution of 1:5 to produce a suspension,
d) filtering the suspension to produce a filtrate, and
e) centrifuging said filtrate to produce a residue of fibroblast-like cells.

2. The method according to claim 1, wherein in step c) said fermentation is carried out for 8-10 hours at 37°C.

3. Method for producing endothelial cells and fibroblast-like cells comprising the steps according to claim 1 or 2, **characterized in that** prior to step b) additionally endothelial cells are isolated from the umbilical cord vein in the process of preparing the umbilical cord fragment, wherein said isolation of said endothelial cells comprises the following steps:
i) filling up said umbilical cord vein with an enzyme solution comprising dispase and incubating it to produce a cell suspension,
ii) removing the umbilical cord vein content cell suspension and centrifuging said suspension to produce a residue of endothelial cells.

4. The method according to claim 3, wherein in step i) said incubation is carried out for 15 minutes.

## Patentansprüche

1. Verfahren zur Herstellung von Fibroblasten-ähnlichen Zellen aus der Nabelschnur von Neugeborenen, wobei das Verfahren die folgenden Schritte umfasst:
a) Vorbereitung eines Fragments der menschlichen Nabelschnur,
b) Homogenisieren des vorbereiteten Fragments,
c) Fermentation des Homogenats mit einer Arbeitslösung, die Kollagenase vom Typ I und Kollagenase vom Typ IV in einem Verhältnis der Enzyme von 1: 1 bei einem Anteil des Homogenats zu der Enzymarbeitslösung von 1:5 enthält, um eine Suspension herzustellen,
d) Filtrieren der Suspension, um ein Filtrat zu erzeugen, und
e) Zentrifugieren des Filtrats, um einen Rest von Fibroblasten-ähnlichen Zellen zu erzeugen.

2. Verfahren nach Anspruch 1, wobei in Schritt c) die Fermentation für 8-10 Stunden bei 37°C durchgeführt wird.

3. Verfahren zur Herstellung von Endothelzellen und Fibroblasten-ähnlichen Zellen umfassend die Schritte gemäß Anspruch 1 oder 2, **dadurch gekennzeichnet, dass** vor Schritt b) zusätzlich Endothelzellen aus der Nabelschnurvene im Prozess der Herstellung des Nabelschnurfragments isoliert werden, wobei die Isolierung der Endothelzellen die folgenden Schritte umfasst:
i) Auffüllen der Nabelschnurvene mit einer Dispase enthaltenden Enzymlösung und Inkubation um eine Zellsuspension herzustellen,
ii) Entfernen der Nabelschnurveneninhalt-Zellsuspension und Zentrifugieren der Suspension, um einen Rest von Endothelzellen zu erzeugen.

4. Verfahren nach Anspruch 3, wobei in Schritt i) besagte Inkubation für 15 Minuten durchgeführt wird.

## Revendications

1. Procédé de production de cellules semblables à des fibroblastes à partir du cordon ombilical de nouveau-nés, ledit procédé comprenant les étapes suivantes :
a) préparation d'un fragment du cordon ombilical humain,
b) homogénéisation du fragment préparé,
c) fermentation de l'homogénéisat avec une solution de travail contenant une collagénase de type I et une collagénase de type IV selon un rapport des enzymes de 1:1, dans une proportion de l'homogénéisat contre la solution de travail enzymatique de 1:5 pour produire une suspension,
d) filtrage de la suspension pour produire un filtrat, et
e) centrifugation dudit filtrat pour produire un résidu de cellules semblables à des fibroblastes.

2. Procédé selon la revendication 1, dans lequel à l'étape c) ladite fermentation est réalisée pendant 8 à 10 heures à 37 °C.

3. Procédé de production de cellules endothéliales et de cellules semblables à des fibroblastes, comprenant les étapes selon la revendication 1 ou 2, **caractérisé en ce qu'**avant l'étape b), des cellules endothéliales sont en outre isolées à partir de la veine du cordon ombilical au cours du processus de préparation du fragment de cordon ombilical, dans lequel ledit isolement desdites cellules endothéliales comprend les étapes suivantes :
i) remplissage de ladite veine du cordon ombilical avec une solution enzymatique comprenant une dispase et sa mise en incubation pour produire une suspension cellulaire,
ii) retrait de la suspension cellulaire présente dans la veine du cordon ombilical et centrifugation de ladite suspension pour produire un résidu de cellules endothéliales.

4. Procédé selon la revendication 3, dans lequel à l'étape i) ladite incubation est réalisée pendant 15 minutes.
